# EUROPÄISCHE PATENTSCHRIFT

(11) **EP 2 445 430 B1**
(45) Veröffentlichungstag und Bekanntmachung des Hinweises auf die Patenterteilung: **21.05.2014**
(21) Anmeldenummer: 10726963.1
(22) Anmeldetag: 23.06.2010
(51) Int. Cl.: A61B 17/80

(54) **REPOSITIONSGERÄT FÜR DIE BEHANDLUNG DISTALER RADIUSFRAKTUREN MITTELS EINER T-FÖRMIGEN OSTEOSYNTHESEPLATTE**
REPOSITIONING DEVICE FOR THE HANDLING OF DISTAL RADIUS FRACTURES BY MEANS OF A T-SHAPED OSTEOSYNTHESIS PLATE
APPAREIL DE REPOSITIONNEMENT POUR LE TRAITEMENT DE FRACTURES DISTALES DU RADIUS AU MOYEN D'UNE PLAQUE D'OSTEOSYNTHESE EN T

(30) Priorität: 25.06.2009 DE 102009030622
(43) Veröffentlichungstag der Anmeldung: 02.05.2012
(73) Patentinhaber: Universität Rostock, 18055 Rostock (DE)
(72) Erfinder: GRADL, Georg, 18211 Boergerende (DE); STEDTFELD, Hans-Werner, 18057 Rostock (DE)
(74) Vertreter: Wablat Lange Karthaus
(86) Internationale Anmeldenummer: PCT/EP2010/058910
(87) Internationale Veröffentlichungsnummer: WO 2010/149702

(56) Entgegenhaltungen:
- EP-A2- 1 308 135
- DE-A1- 3 912 703

## Beschreibung

Die Erfindung bezieht sich auf ein Repositionsgerät für die Behandlung distaler Radiusfrakturen mittels einer T-förmigen Osteosyntheseplatte gemäß dem Oberbegriff des Patentanspruches 1.

Zur Behandlung von Frakturen des körperfernen Unterarmes (Speiche, Radius) ist die Verwendung winkelstabiler Platten bekannt. Ein Problem hierbei ist das Erzielen einer adäquaten Reposition der Frakturfragmente. Das Ergebnis soll sein, die menschliche Anatomie vor dem Sturz wiederherzustellen.

Für die körperferne, distale Speiche (Radius) ist die anatomische Stellung in der Frontalebene mit einem Winkel von 25° Steilheit und ca. 1 - 5 mm Überlänge gegenüber der Elle (Ulna) auf Höhe des distalen Radioulnargelenkes maßgebend. In der Saggitalebene ist die Gelenkfläche des Radius um ca. 10° nach palmar (hohlhandseits) gekippt. Bei einer typischen Fraktur des Radius kommt es zu einer Dislokation des distalen Frakturfragmentes aus dieser anatomischen Position heraus um 20 - 50° nach handrückenwärts (dorsal). Diese Dislokation muss der behandelnde Chirurg aufheben. Das gelingt aber meist nicht vollständig. Mit einer herkömmlichen Operation wird ein Winkel von 0° bis 5°, in vielen Fällen lediglich ein Repositionswinkel von 0° erreicht. In diesem Fall wird das distale Frakturfragment in der Saggitalebene lediglich senkrecht auf den Radiusschaft eingestellt.

Aus der US 2005/0065522 A1 ist eine T-förmige Osteosyntheseplatte vorbekannt. Bei deren Verwendung zur Osteotomie wird der kurze Schenkel mittels Knochenschrauben am distalen Ende des Knochens, dem späteren Fraktursegment, angeschraubt, wobei der lange Schenkel unter einem Winkel vom körpernahen, proximalen Knochen absteht. Dann wird der Knochen proximal zum distalen Ende abgesägt und die Platte wird mittels ihres langen Schenkels um den Winkel zum körpernahen Knochen geschwenkt und mittels durch den langen Schenkel in den körpernahen, proximalen Knochen eingebrachter Knochenschrauben am körpernahen, proximalen Knochen angeschraubt.

Aus der EP 1 308 135 A2 ist eine T-förmige Osteosyntheseplatte bekannt, die aus einem langen Schenkel und einem winklig zu diesem angeordneten kurzen Schenkel gebildet ist, der zur Fixierung mit Schrauben auf dem dislozierten Fragment vorgesehen ist, wobei der lange Schenkel unter einem Winkel vom körpernahen, proximalen Knochen absteht. Nach dem Absägen des Knochens proximal zum distalen Ende wird die Platte mittels ihres langen Schenkels um den Winkel zum körpernahen Knochen geschwenkt und mittels Knochenschrauben am körpernahen, proximalen Knochen angeschraubt. Zusätzlich ist am Plattenkörper ein quaderförmiger Platz- oder Abstandshalter verschiebbar befestigt, der zum Einsetzen in die Öffnung des Osteotmie-Keilschnittes dient.

Die bekannte T-förmige Osteosyntheseplatte ist nicht nur im Zusammenhang mit einer Osteotomie, sondern auch im Zusammenhang mit Knochenbrüchen anwendbar, wie es einleitend beschrieben worden ist. Bei einer typischen Fraktur des körperfernen, distalen Radius muß die Dislokation des distalen Frakturfragmentes vom behandelnden Chirurgen aufgehoben werden. Dies gelingt mit der vorbekannten T-förmigen Osteosyntheseplatte jedoch meist nicht vollständig.

Der Erfindung liegt von daher die Aufgabe zugrunde, das Repositionsgerät für die Behandlung distaler Radiusfrakturen mittels einer T-förmigen Osteosyntheseplatte dahingehend zu verbessern, dass die erforderliche Kippung der Gelenkfläche nach palmar der eingesetzten Osteosyntheseplatte winkelgenau durchgeführt werden kann.

Zur Lösung dieser Aufgabe sieht die Erfindung ein Repositionsgerät vor, bei dem die dem proximalen Knochen oder Radiusschaft zugewandte untere Fläche des langen, proximalen Schenkels der T-förmigen Platte mit einem Abstandshalter in Form eines Keils versehen ist, um den langen Schenkel unter einem bestimmten Winkel zum körpernahen, proximalen Knochen zu halten.

Hierfür wird der Abstandshalter unter dem langen Schenkel der T-förmigen Platte zur Erzielung des erforderlichen Winkels eingestellt. Wenn der lange Schenkel dann unter Zwischenlage des keilförmigen Abstandshalters mit seiner unteren Fläche auf den körpernahen, proximalen Knochen aufgelegt wird - der kurze Schenkel der T-förmigen Platte liegt bereits mit seiner Unterseite auf dem disloziierten Frakturfragment auf - wird die T-förmige Platte zunächst distal mit Knochenschrauben fixiert. Der lange Schenkel der T-förmigen Platte liegt also auf dem proximalen Knochen nicht direkt auf, sondern steht einige Millimeter ab. Somit ergibt sich ein Winkel zwischen 5° und 20° zwischen dem in Neutralstellung (Neutral in Bezug auf die Schaftachse des Knochens) fixierten distalen Frakturfragment und der T-förmigen Platte. Werden nun der keilförmige Abstandshalter vom Chirurgen entfernt und die T-förmige Platte mit dem langen Schenkel auf den proximalen Knochen gedrückt, wird das an der T-förmigen Platte befestigte distale Frakturfragment um den Winkel des zuvor eingebrachten keilförmigen Abstandshalters nach palmar gekippt. Somit wird auf einfache Weise die Anatomie des distalen Radius mit 10° palmarer Kippung in der Saggitalebene wiederhergestellt, d. h. die restlichen 10° Kippung der Gelenkfläche nach palmar werden zusammen mit der T-förmigen Platte wieder erreicht.

In weiterer Ausbildung der Erfindung ist der keilförmige Abstandshalter bzw. der Keil auf der dem körpernahen, proximalen Knochen zugewandten unteren Fläche des langen Schenkels angeordnet. Der Keil muß zur Erzielung eines bestimmten, gewünschten Winkels zwischen dem langen Schenkel und dem proximalen Knochen vor dem Einsetzen in seiner Größe an den gewünschten Winkel angepasst werden.

Erfindungsgemäß weist der Keil einen Winkel von 5° bis 20° zwischen seinen auf dem körpernahen, proximalen Knochen und der diesem zugewandten unteren Fläche des langen Schenkels zur Anlage kommenden Keilflächen auf. Ferner ist der Keil auf der dem körpernahen, proximalen Knochen zugewandten Auflageseite des langen Schenkels lösbar befestigbar. Mit dem erfindungsgemäßen Keil wird eine anatomische Reposition von mindestens ca. 15° erreicht.

In noch weiterer Ausbildung der Erfindung ist die dem proximalen Knochen oder Radiusschaft zugewandte untere Fläche des langen, proximalen Schenkels der T-förmigen Platte mit einem Abstandshalter bzw. Distanzelement in Form einer im langen Schenkel schraubbaren Distanzschraube bzw. eines Schraubstift mit einer Anlagefläche für den körpernahen, proximalen Knochen versehen, um den langen Schenkel unter einem Winkel zum körpernahen, proximalen Knochen zu halten. Die in dem langen Schenkel schraubbare Distanzschraube ermöglicht eine leichte Anpassung des Winkels zwischen dem langen Schenkel und dem proximalen Knochen an die Erfordernisse des Einsatzfalles.

Das erfindungsgemäße Repositionsgerät für die Behandlung distaler Radiusfrakturen mittels einer T-förmigen Osteosyntheseplatte wird nachfolgend an Hand mehrerer Ausführungsformen näher erläutert.

Es zeigt:
- Fig. 1a: den distalen Radius eines distalen Radioulnargelenkes in der Frontalebene,
- Fig. 1b: den distalen Radius nach Fig. 1a in der Sagittalebene,
- Fig. 2a: eine distale Radiusfraktur mit Dislokation des distalen Frakturfragmentes nach dorsal,
- Fig. 2b: die distale Radiusfraktur nach Fig. 2a postoperativ mit einer T-förmigen Osteosyntheseplatte gemäß dem Stand der Technik,
- Fig. 3a: die distale Radiusfraktur nach Fig. 2a mit einer T-förmigen Osteosyntheseplatte und mit einem Abstandshalter in Form eines Keils unter dem langen Schenkel der T-förmigen Osteosyntheseplatte gemäß der ersten Ausführungsform der Erfindung,
- Fig. 3b: die distale Radiusfraktur nach Fig. 3a postoperativ nach dem Entfernen des Keils unter dem langen Schenkel der T-förmigen Osteosyntheseplatte in der ersten Ausführungsform der Erfindung,
- Fig. 4a: die aus der zweiten Ausführungsform nach Fig. 3a abgewandelte zweite Ausführungsform der Erfindung mit einem verschraubten Keil unter dem langen Schenkel der T-förmigen Platte,
- Fig. 4b: die aus der dritten Ausführungsform nach Fig. 4a abgewandelte vierte Ausführungsform der Erfindung mit einer Distanzschraube als Abstandshalter und
- Fig. 5: a bis e weitere Abstandshaltern in Form eines Keils, eines Zylinders, eines Quaders, einer Distanzschraube und eines Schraubstiftes. Die Abstandhalter der Fig. 5b und 5c (der Quader und der Zylinder) fallen nicht unter den Schutzumfang von Anspruch 1.

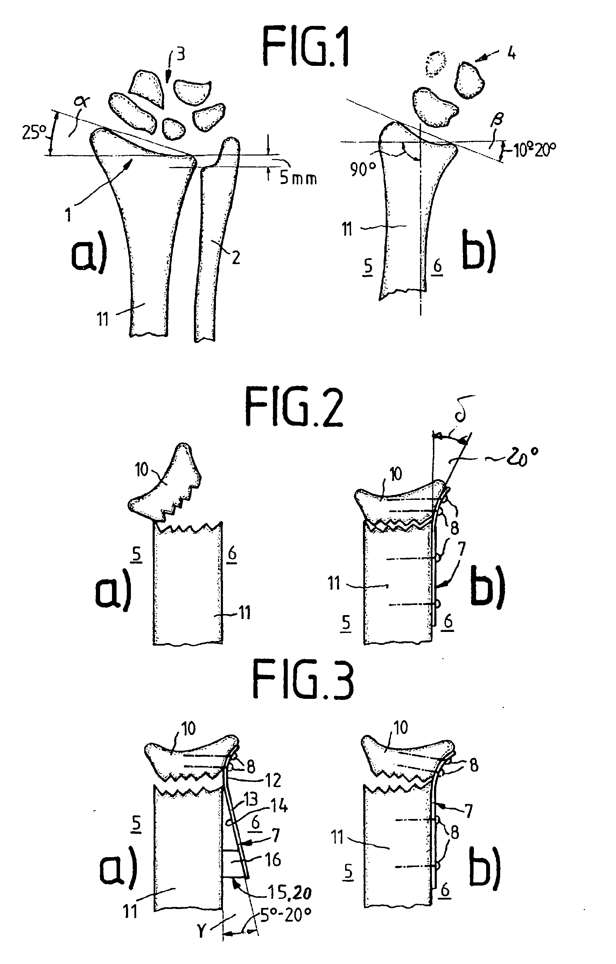

Die Fig. 1a und 1b zeigen den körperfernen Unterarm (Speiche, Radius) eines Patienten mit dem Radius 1, der Elle (Ulna) 2, der Mittelhand 3 und den Mittelhandknochen 4. Das Problem bei der Behandlung einer Fraktur des körperfernen Unterarmes (Fig. 2a) ist das Erzielen einer adäquaten Reposition der Frakturfragmente 10. Ziel ist es, die menschliche Anatomie vor einem Sturz (Fig. 1 a, 1b) nach einer Fraktur (Fig. 2a) wieder herzustellen. Für die körperferne, distale Speiche bzw. den Radius 1 ist die anatomische Stellung in der Frontalebene (Fig. 1a) mit einem Winkel α von 25° Steilheit und ca 1 - 5 mm Überlänge (Fig. 1a) gegenüber der Elle (Ulna) 2 auf der Höhe des distalen Radioulnargelenkes maßgebend. In der Sagittalebene gemäß Fig. 1b ist die Gelenkfläche des Radius 1 um einen Winkel β von ca. 10° nach palmar (hohlhandseits) gemäß Bezugsziffer 6 in Fig. 1b und 2a gekippt. Bei einer typischen Fraktur des Radius 1 kommt es zu einer Dislokation des distalen Frakturfragmentes 10 aus dieser anatomischen Position heraus um 20 bis 50° nach handrückenwärts (dorsal) gemäß Bezugsziffer 5 in Fig. 2a. Diese Dislokation muß der behandelnde Chirurg aufheben. Das gelingt aber meist nicht vollständig. In vielen Fällen werden lediglich eine Reposition von O° erreicht. In diesem Fall kann das distale Frakturfragment 10 in der Sagittalebene gemäß Fig. 2b lediglich senkrecht auf den Radiusschaft 11 eingestellt werden.

Diese vorbekannte Reposition erfolgt mittels einer T-förmigen Osteosyntheseplatte 7, kurz T-förmige Platte 7, deren kurze und lange Schenkel 12, 13 gemäß Fig. 2b am Frakturfragment 10 bzw. am Radiusschaft 11 mittels Öffnungen 9 in der T-förmigen Platte 7 durchgreifender Knochenschrauben 8 palmar gemäß Bezugsziffer 6 verschraubt werden, wobei der Radius 1 die in Fig. 2b gezeigte Position bezüglich des Radiusschaftes 11 einnimmt. Der Biegungswinkel δ zwischen dem kurzen und dem langen Schenkel 12 bzw. 13 beträgt etwa 20° (Fig. 2b).

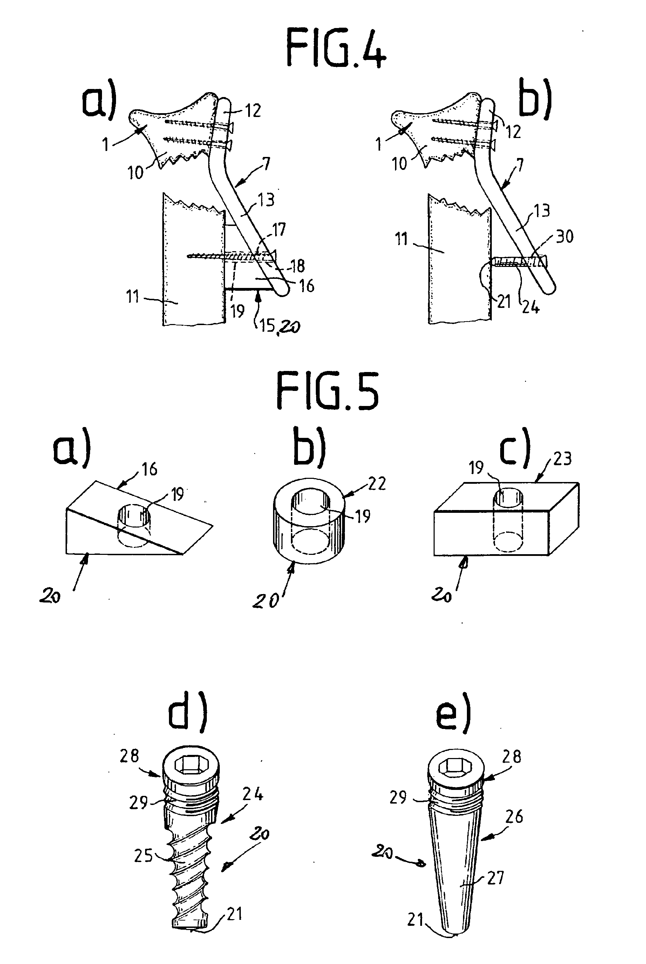

Um dem Chirurgen eine Erleichterung zu verschaffen, die restlichen 10° Kippung der Gelenkfläche nach palmar gemäß Bezugsziffer 6 zusammen mit der eingesetzten T-förmigen Platte 7 zu erreichen, ist gemäß der vorliegenden Erfindung die dem proximalen Knochen oder Radiussschaft 11 zugewandte untere Fläche 14 des langen, proximalen Schenkels 13 mit einem Abstandshalter 15 in Form eines Keils 16 versehen, um mittels dessen Keilflächen den langen Schenkel 13 unter einem bestimmten Winkel γ von zwischen 5° und 20° zu halten. Der Keil 16 kann insbesondere mittels einer Knochenschraube 17, die Bohrungen 18 und 19 im langen Schenkel 13 und im Keil 16 durchdringt, gehalten sein (Fig. 4a). Wenn die vorgeformte T-Platte 7 auf den Knochen bzw. Radiussschaft 11 aufgelegt wird, nachdem der kurze Schenkel 12 der Platte 7 bereits mittels Knochenschrauben 8 distal auf dem dislozierten Frakturfragment 10 befestigt ist, liegt der lange Schenkel 13 der T-Platte 7 also auf dem proximalen Knochen oder Radiusschaft 11 nicht direkt auf, sondern steht, je nach der Größe des Winkels γ, um einige Millimeter ab. Somit ergibt sich ein Winkel γ zwischen 5° und 20° zwischen den in Neutralstellung (Neutral in bezug auf die Schaftachse des proximalen Knochens oder Radiusschaft 11) fixierten distalen Fakturfragment 10 und der T-Platte 7. Wird nun der Keil 16 vom Chirurgen entfernt und die T-Platte 7 mit ihrem langen Schenkel 13 auf den proximalen Knochen oder Radiusschaft 11 aufgedrückt, wird das am kurzen Schenkel 12 der T-förmigen Platte 7 befestigte distale Frakturfragment 10 um den Winkel γ des zuvor eingebrachten und nun entfernten Keils 16 nach palmar (Bezugsziffer 6) gemäß Fig. 3b gekippt. Somit wird auf einfache Weise die Anatomie des distalen Radius 1 mit 10° palmarer Kippung in der Saggitalebene (Fig. 3b) wieder hergestellt. Der Abstandshalter 15, hier der Keil 16, führt zu einer anatomischen Reposition von mindestens ca. 15°.

Der Abstandshalter 15 bildet ein auf der dem körpernahen, proximalen Knochen oder Radiusschaft 11 zugewandten unteren Fläche 14 des langen Schenkels 13 angeordnetes Distanzelement 20. Dieses kann gemäß Fig. 3a und 4a ein Keil 16 sein, der mittels einer die Bohrungen 18 und 19 im langen Schenkel 13 der T-förmigen Platte 7 bzw. des Keils 16 (Fig. 5a) durchdringenden Knochenschraube 17, die in den proximalen Knochen oder Radiusschaft 11 eingeschraubt wird, gemäß Fig. 4a befestigt wird. Das Distanzelement 20 kann aber auch gemäß Fig. 5b ein Zylinder 22 oder gemäß Fig. 5c ein Quader 23 sein, die jeweils wie der Keil 16 mit Bohrungen 19 zur Fixierung versehen sind. Diese zwei Alternativen fallen jedoch nicht unter den Schutzumfang der Ansprüche.

Das Distanzelement 20 kann auch gemäß Fig. 4b i. V. m. Fig 5d oder 5e eine Distanzschraube 24 mit einem Schaftgewinde 25 oder ein Schraubstift 26 mit glattem Schaft 27 sein, die jeweils mit Anlageflächen 21 für den körpernahen, proximalen Knochen bzw. Radiusschaft 11 versehen sind. Sowohl die Distanzschraube 24 als auch der Schraubstift 26 besitzen am Kopf 28 ein Gewinde 29, mit welchem diese in eine Gewindebohrung 30 im langen Schenkel 13 der T-förmigen Platte 7 einschraubbar und mit dieser verbindbar sind.

Mittels der Distanzschraube 24 bzw. des Schraubstiftes 26 verschiedener Länge können beliebige Abstände zwischen dem langen Schenkel 13 der T-förmigen Platte 7 und dem proximalen Knochen oder Radiusschaft 11 und damit beliebige Winkel γ eingestellt werden. Damit ist ersichtlich, dass ein Abstandshalter 16 als Distanzelement 20 vorgesehen ist, der bzw. das in nicht notwendiger Weise ein fester Keil 16 ist, sondern auch andere geometrische Figuren verwendbar sind.

Das Verfahren zur Behandlung distaler Radiusfrakturen mittels der T-förmigen Osteoysntheseplatte 7 des vorbeschriebenen Repositionsgerätes ist dadurch gekennzeichnet, dass der kurze, distale Schenkel 13 der T-förmigen Platte 7 am distalen Frakturfragment 10 fest angeschraubt wird, dass der lange, proximale Schenkel 13 der T-förmigen Platte 7 gegenüber dem proximalen, körpernahen Knochen oder Radiusschaft 11 mittels des Abstandshalters 15 oder Distanzelementes 20 unter einem bestimmten Winkel γ zwischen 5° und 20° auf Abstand gehalten wird und dass nach dem Entfernen des Abstandshalters 15 oder Distanzelementes 20 der lange Schenkel 13 der T-förmigen Platte 7 auf den proximalen Knochen oder Radiusschaft 11 gedrückt und dort mittels Knochenschrauben 8 befestigt wird, wodurch das an der T-förmigen Platte 7 befestigte distale Frakturfragment 10 um den eingestellten Winkel γ nach palmar gemäß Bezugsziffer 6 gekippt wird (Fig. 3 a , 3b).

Die anatomische Anpassung der T-förmigen Platte 7, die eine Angulation bzw. einen Winkel δ von etwa 20° des kurzen Schenkels 12 der T-förmigen Platte 7 gegenüber dem langen Schenkel 13 aufweist, schließt aus, dass diese Vorbiegung allein auch automatisch zu einer entsprechenden Fraktur-reposition führt. Mit den im Stand der Technik vorbekannten winkligen T-förmigen Oseosyntheseplatten wird, wie es einleitend bereits ausgeführt worden ist, eher die 0°-Stellung der Gelenkfläche erreicht. Durch die erfindungsgemäße Ausgestaltung der angulierten T-förmigen Platte 7, welche Angulierung sich aus der Plattenvorbiegung um den Winkel δ und der Anwendung eines Abstandshalters 15 bzw. Distanzelementes 20, z. B. in Form des Keils 16 automatisch ergibt, wird die Fraktur in die notwendige Kippung "gezwungen". Mit der Angulation der T-förmigen Platte 7 allein ist das nur möglich, wenn die T-förmige Platte 7 bei der Operation irgendwie "in der Luft gehalten wird". Dies birgt die Gefahr in sich, dass dann die T-förmige Platte 7 nicht orthograd auf dem Knochenschaft bzw. Radiusschaft 11 zu liegen kommt, sondern seitlich versetzt wird. Der Chirurg kann unmöglich eine händisch in der Luft gehaltene T-förmige Platte 7 mit der geforderten Präzision später dem Knochen bzw. Radiusschaft 11 annähern. Außerdem wird hierdurch auch kein definierter Abstand eingehalten, welcher später eine vorhersehbare Kippung des Frakturfragmentes 10 erlaubt. Diese rein "händische" Präzision ist nicht ausreichend und wird durch das erfindungegemäße Repositionsgerät und das mit diesem durchgeführte Verfahren grundlegend verändert.

Die T-förmige Osteosyntheseplatte 7 allein ist kein Repositionsgerät für die Behandlung distaler Radiusfrakturen, sondern eine reine Abstützplatte, welche durch ihre Angulation der Anatomie angepasst ist und auf einen durch andere Fixierelemente wie Kirschnerdrähte vorreponierten und bereits anatomisch vorfixierten Knochen aufgelegt wird und durch ihre Angulation diesen dann bei der Fixierung nicht wieder verschiebt.

Es folgt einer Gegenüberstellung des zeitlichen Vorgehens bei
A: einer normalen Operation mit einer T-förmigen Osteosyntheseplatte gemäß dem Stand der Technik:
   1. Frakturdarstellung,
   2. Einrichten - also Reponieren-
   3. Halten der Reposition temporär mit Drähten,
   4. Erzielen einer gewünschten Frakturstellung,
   5. wenn die gewünschte Frakturstellung erreicht ist, Anbringen der T-förmigen Osteosyntheseplatte,
   6. Fixieren der T-förmigen Platte.
   mit
B: dem zeitlichen Vorgehen mit einer Osteosyntheseplatte in der ersten Ausführungsform der Erfindung mit einem Keil:
   1. Frakturdarstellung,
   2. Einrichten, also Reponieren,
   3. Halten der Repositon mit Drähten,
   4. Dann - unabhängig von der erzielten Reposition - Anbringen der T-förmigen Platte mit dem Keil,
   5. Fixieren der Platte mit dem Repositionskeil am Knochen- bzw. Radiusschaft,
   6. Fixieren der Fraktur,
   7. Entfernen des Keils und jetzt - durch Annähern der Platte an den Knochen- Erzielen der gewünschten Reposition.

Diese Gegenüberstellung der Repositionierverfahren gemäß dem Stand der Technik und gemäß der Erfindung zeigt auf, dass das zeitliche Vorgehen bei einer normalen Operation mit dem zeitlichen Vorgehen mit einem Repositionsgerät gemäß der Erfindung nur in den Schritten 1 bis 3 übereinstimmt. Dann nimmt das Verfahren bei Anwendung des erfindungsgemäßen Repositionsgerätes mit den Schritten 4 bis 7 einen anderen Verfahrensweg als das vorbekannte Verfahren dann mit den Schritten 4 bis 6.

### Bezugszeichenliste

- 1: Radius
- 2: Elle (Ulna)
- 3: Mittelhand
- 4: Mittelhandknochen
- 5: dorsal
- 6: palmar
- 7: Platte
- 8: Knochenschraube
- 9: Öffnung
- 10: Frakturfragment
- 11: Radiusschaft
- 12: kurzer Schenkel
- 13: langer Schenkel
- 14: untere Fläche
- 15: Abstandshalter
- 16: Keil
- 17: Knochenschraube
- 18: Bohrung
- 19: Bohrung
- 20: Distanzelement
- 21: Anlagefläche
- 22: Zylinder
- 23: Quader
- 24: Distanzschraube
- 25: Schaftgewinde
- 26: Schraubstift
- 27: Schaft
- 28: Kopf
- 29: Gewinde
- 30: Gewindebohrung

## Patentansprüche

1. Repositionsgerät für die Behandlung distaler Radiusfrakturen mittels einer T-förmigen Osteosyntheseplatte, aus einem langen Schenkel und einem zu diesem T-förmig und winklig angeordneten kurzen Schenkel, wobei der kurze, distale Schenkel eine am distalen Frakturfragment zur Anlage kommende Fläche und eine Anzahl von Öffnungen für Knochenschrauben aufweist und der lange, proximale Schenkel zur Anlage am körpernahen, proximalen Knochen vorgesehen ist, wobei die dem proximalen Knochen oder Radiusschaft (11) zugewandte untere Fläche (14) des langen, proximalen Schenkels (13) der T-förmigen Platte (7) mit einem Abstandshalter (15) versehen ist, um den langen Schenkel (13) unter einem bestimmten Winkel (γ) zum körpernahen, proximalen Knochen (11) zu halten, **dadurch gekennzeichnet, dass** der Abstandhalter ein Keil (16), eine im langen Schenkel (13) schraubbare Distanzschraube (24) oder ein im langen Schenkel (13) schraubbarer Schraubstift (26) mit einer Anlagefläche (31) für den proximalen Knochen (11) ist.

2. Repositionsgerät nach Anspruch 1, **dadurch gekennzeichnet, dass** der Keil (16) auf der dem körpernahen, proximalen Knochen (11) zugewandten unteren Fläche (14) des langen Schenkels (13) angeordnet ist.

3. Repositionsgerät nach Anspruch 1 oder 2, **dadurch gekennzeichnet, dass** der Keil (16) einen Winkel (γ) von 5 bis 20 ° zwischen seinen auf dem körpernahen, proximalen Knochen (11) und der diesem zugewandten unteren Fläche (14) des langen Schenkels (13) zur Anlage kommenden Keilflächen aufweist.

4. Repositionsgerät nach einem der Ansprüche 1 bis 3, **dadurch gekennzeichnet, dass** der Keil (16) auf der dem körpernahen, proximalen Knochen (11) zugewandten unteren Fläche (14) des langen Schenkels (13) lösbar befestigbar ist.

5. Repositionsgerät nach Anspruch 1, **dadurch gekennzeichnet, dass** die Distanzschraube (24) ein Kopfgewinde (29) und einen glatten Schaft (27) aufweist.

## Claims

1. A repositioning device for the treatment of distal radius fractures by means of a T-shaped osteosynthesis plate comprising a long leg and a short leg disposed in a T-shape and at an angle with respect to said long leg, wherein the short, distal leg has a face bearing against the distal fracture fragment and a number of openings for bone screws and the long, proximal leg is provided to bear against the proximal bone close to the body, wherein the lower face (14) of the long, proximal leg (13) of the T-shaped plate (7) facing the proximal bone or radius shaft (11) is provided with a spacer (15) to keep the long leg (13) at a specific angle (γ) to the proximal bone (11) close to the body, **characterized in that** the spacer is a wedge (16), a distancing screw (24) that can be screwed in the long leg (13) or a threaded pin (26) that can be screwed in the long leg (13) having a bearing face (31) for the proximal bone (11).

2. The repositioning device according to claim 1, **characterized in that** the wedge (16) is disposed on the lower face (14) of the long leg (13) facing the proximal bone (11) close to the body.

3. The repositioning device according to claim 1 or 2, **characterized in that** the wedge (16) forms an angle (γ) of 5 to 20° between its wedge faces bearing against the proximal bone (11) close to the body and the lower face (14) of the long leg (13) facing said proximal bone.

4. The repositioning device according to one of the claims 1 to 3, **characterized in that** the wedge (16) can be detachably fastened on the lower face (14) of the long leg (13) facing the proximal bone (11) close to the body.

5. The repositioning device according to claim 1, **characterized in that** the distancing screw (24) has a head thread (29) and a smooth shank (27).

## Revendications

1. Appareil de repositionnement pour le traitement de fractures distales du radius au moyen d'une plaque d'ostéosynthèse en T, composée d'une branche longue et d'une branche courte disposée en T et en angle par rapport à celle-ci et, sachant que la branche distale courte présente une face venant en appui contre le fragment distal de la fracture et plusieurs orifices pour des vis à os, et la branche proximale longue est prévue pour venir en appui contre l'os proximal proche du corps, sachant que la face inférieure (14) de la branche proximale longue (13) de la plaque en T (7) tournée vers l'os proximal ou le corps du radius, est dotée d'un écarteur (15) destiné à maintenir la branche longue (13) dans un angle déterminé (γ) par rapport à l'os proximal proche du corps (11), **caractérisé en ce que** l'écarteur est une cale (16), une vis d'écartement (24) pouvant être vissée dans la branche longue (13) ou une tige filetée (26) pouvant être vissée dans la branche longue (13) avec une face d'appui (31) pour l'os proximal (11).

2. Appareil de repositionnement selon la revendication 1, **caractérisé en ce que** la cale (16) est disposée sur la face inférieure (14) de la branche longue (13) tournée vers l'os proximal proche du corps (11).

3. Appareil de repositionnement selon la revendication 1 ou 2, **caractérisé en ce que** la cale (16) présente un angle (γ) de 5 à 20° entre ses surfaces venant s'appuyer contre l'os proximal proche du corps (11) et contre la face inférieure (14) de la branche longue (13) tournée vers celui-ci.

4. Appareil de repositionnement selon l'une des revendications 1 à 3, **caractérisé en ce que** la cale (16) est fixée de façon séparable sur la face inférieure (14) de la branche longue (13) tournée vers l'os proximal proche du corps (11).

5. Appareil de repositionnement selon la revendication 1, **caractérisé en ce que** la vis d'écartement (24) présente une tête filetée (29) et une tige lisse (27).
